# EUROPÄISCHE PATENTSCHRIFT

(11) **EP 2 051 616 B1**
(45) Veröffentlichungstag und Bekanntmachung des Hinweises auf die Patenterteilung: **14.06.2017**
(21) Anmeldenummer: 07786555.8
(22) Anmeldetag: 03.08.2007
(51) Int. Cl.: A61B 3/10, A61B 3/18

(54) **VORRICHTUNG ZUR INDIVIDUELLEN THERAPIEPLANUNG UND POSITIONSGENAUEN MODIFIKATION EINES OPTISCHEN ELEMENTS**
APPARATUS FOR INDIVIDUAL THERAPY PLANNING AND POSITIONALLY ACCURATE MODIFICATION OF AN OPTICAL ELEMENT
DISPOSITIF POUR LA PLANIFICATION THÉRAPEUTIQUE INDIVIDUELLE ET LA MODIFICATION EXACTE DE POSITION D'UN ÉLÉMENT OPTIQUE

(30) Priorität: 07.08.2006 DE 102006036800
(43) Veröffentlichungstag der Anmeldung: 29.04.2009
(73) Patentinhaber: Carl Zeiss Meditec AG, 07745 Jena (DE)
(72) Erfinder: DICK, Manfred, 07926 Gefell (DE); REICH, Matthias, 07749 Jena (DE); ZIMARE, Diego, 07952 Pausa (DE)
(74) Vertreter: DTS Patent- und Rechtsanwälte Schnekenbühl und Partner mbB
(86) Internationale Anmeldenummer: PCT/EP2007/006898
(87) Internationale Veröffentlichungsnummer: WO 2008/017428

(56) Entgegenhaltungen:
- WO-A-03/039356
- WO-A-2006/074469
- US-A- 5 920 373
- US-A1- 2003 220 629
- US-A1- 2004 199 149
- DICK H B ET AL: "[Dynamic aberrometry during accommodation of phakic eyes and eyes with potentially accommodative intraocular lenses]" DER OPHTHALMOLOGE : ZEITSCHRIFT DER DEUTSCHEN OPHTHALMOLOGISCHEN GESELLSCHAFT NOV 2002, Bd. 99, Nr. 11, November 2002 (2002-11), Seiten 825-834, XP002470325 ISSN: 0941-293X

## Beschreibung

Die Erfindung betrifft eine Vorrichtung zur individuellen Therapieplanung und zur positionsgenauen Modifikation eines optischen Elements. Insbesondere betrifft die Erfindung eine Navigationsvorrichtung zur optischen Analyse und Modifikation eines optischen Elements, insbesondere eine Navigationsvorrichtung zur optischen Analyse und Modifikation eines gealterten humanen Auges zur Behandlung der Presbyopie.

In der Ophtalmologie ist es bekannt, die Hornhaut durch Operation von Gewebe zu formen und damit Myopie, Hyperopie und Astigmatismus zu korrigieren. Hierfür werden Laserstrahlen beispielsweise eines ArF-Excimerlasers eingesetzt, dessen Impuls zur Strahlung eine Wellenlänge von 193 nm hat. Neben der laserchirurgischen refraktiven Korrektur der Kornea sind Verfahren zur Therapie der Augenlinse zur Behandlung der Presbyopie beschrieben worden. Hierbei wird versucht, die erhärtete Linse durch geeignete Schnitte oder Blasenfelder wieder in einen Zustand der besseren Verformbarkeit durch den Kapselsack bzw. Ziliarmuskel zu versetzen. Damit soll prinzipiell die Akkomodationsfähigkeit der Linse teilweise regeneriert werden.

In der WO2005/070358 werden einfache Schnittgeometrien beschrieben, die in homogenen Materialien die Flexibilität erhöhen sollen. Bei den angegebenen Verfahren bzw. Schnittgeometrien erfolgt jedoch keine Berücksichtigung der individuellen Geometrie oder der inneren Struktur der Augenlinse.

In der WO2006/074469 wird ein Verfahren und eine Vorrichtung für eine gemusterte Plasma-vermittelte Laser Trepanation der Linsenkapsel und dreidimensionale Phako-Segmentation beschrieben.

Aufgabe der vorliegenden Erfindung war es daher eine Vorrichtung und ein Verfahren bereit zu stellen, bei dem die Nachteile des Standes der Technik vermieden werden.

Insbesondere wird die Aufgabe gelöst durch eine erfindungsgemäße Navigationsvorrichtung zur optischen Analyse der inneren Struktur und/oder der individuellen Position eines optischen Elements und zur Modifikation bzw. Bearbeitung des optischen Elements umfassend eine Detektionseinrichtung, wobei die Apertur der Detektionseinrichtung kleiner 0,25 und größer 0,1 ist und eine Bearbeitungseinrichtung, die eingerichtet ist, entlang oder im festen Bezug zu der analysierten inneren Struktur des optischen Elements geführt zu werden.

Durch die Erfindung wird auch ein ophtalmoschirurgisches Gerätesystem unter einer Anleitung zum Umgang mit diesem sehr schonenden und präzisen Präperationen und Entfernung von transparenten biologischen Gewebe bereitgestellt, insbesondere im Auge und im Speziellen in der menschlichen Augenlinse im Rahmen einer refraktiven Laserchirurgie oder Katarakt-OP insbesondere zur Behandlung der Presbyopie durch Regeneration der Akkomodationsfähigkeit des Linsenkörpers erfolgen.

Eine Navigationsvorrichtung ist eine aus mehreren Komponenten bestehende Vorrichtung, die es erlaubt, innerhalb eines optischen Elements einerseits die Struktur zu erkunden bzw. zu analysieren und andererseits an dann festgelegten Stellen dieses optische Element bearbeiten bzw. modifizieren zu können. Mit einer derartigen Navigationsvorrichtung ist es möglich, innerhalb des optischen Elements Strukturen zu erkennen und punktgenau in Abhängigkeit der erkannten Strukturen zu bearbeiten. Besonders bevorzugt handelt es sich um eine ophtalmologische Vorrichtung, die im Rahmen der Augenheilkunde eingesetzt werden kann.

Ein optisches Element ist ein Element, durch das ein optischer Pfad geführt werden kann. Bevorzugt handelt es sich um ein Element mit einer Brechzahl, die unterschiedlich zu der des sie umgebenen Mediums ist. Besonders bevorzugt ist die Brechzahl des optischen Elements größer als die des sie umgebenden Mediums. Das optische Element ist der Bereich, bzw. das Objekt, das durch die Navigationsvorrichtung untersucht bzw. bearbeitet wird. Das optische Element weist bevorzugt innere Strukturen auf. Das optische Element kann beispielsweise mehrschichtig aufgebaut sein und aus Schichten unterschiedlicher Brechzahl bestehen. Es ist auch denkbar, dass das optische Element einschichtig aufgebaut ist. Das optische Element kann auch einen inneren Verlauf von Material unterschiedlicher Brechzahl aufweisen. Das optische Element kann eine optisch wirksame Form wie beispielsweise eine Linsenform aufweisen.

Die optische Analyse ist eine Untersuchung des optischen Elements mit Hilfe einer optischen Einrichtung, bevorzugt mit einer ophtalmologischen Einrichtung, mit der innere Strukturen des optischen Elements bzw. die individuelle Position des optischen Elements bzw. der entsprechenden Strukturen erkannt werden kann. Die inneren Strukturen eines optischen Elements können beispielsweise Grenzflächen bzw. Schichten verschiedener Elemente sein, aus denen das optische Element besteht. Es ist beispielsweise möglich, dass ein Glaskörper aus einem Kern und verschiedenen, zwiebelartig darum gelegten weiteren Schichten besteht. Ebenso ist das menschliche Auge durch den Wachstumsvorgang aus mehreren Schichten aufgebaut, die voneinander als innere Struktur unterschieden werden können. So besteht ein Auge aus einem Embryonalkern, der von einem Fetalkern umgeben ist, der wiederum von einem Adultkern umgeben ist, der wiederum von einer Rinde umgeben ist. Die entsprechenden Grenzflächen zwischen den einzelnen Kernen als innere Strukturen können dann in der optischen Analyse erkannt werden.

Die Navigationsvorrichtung ist auch ausgelegt, um das optische Element zu modifizieren bzw. zu bearbeiten. Hierdurch kann das optische Element in seinen optischen Eigenschaften verändert werden. So ist es möglich, Strukturen innerhalb des optischen Elementes zu verändern, Schnitte einzubringen, das optische Element teilweise zu zerstören und Bereiche aufzulösen, die Brechzahl des Materials des optischen Elements in speziellen Bereichen zu verändern, Schnittgeometrien in das optische Element einzubringen und dadurch die Form und/oder die Beweglichkeit des optischen Elements in sich zu verändern, insbesondere zu erhöhen.

Zur optischen Analyse der inneren Struktur des optischen Elements ist besonders bevorzugt eine Detektionseinrichtung vorgesehen. Diese optische Detektionseinrichtung ist so ausgelegt, dass sie innere Strukturen innerhalb eines optischen Elements erkennen und bevorzugt sichtbar machen kann. Hierzu können für die Bestimmung optisch refraktiver Eigenschaften beispielsweise Aberrometer oder Refraktometer, bevorzugt dynamische Aberrometer beispielsweise auch in Form der jeweiligen Zernikepolynome eingesetzt werden. Besonders bevorzugt kann die dynamische Wellenfrontdiagnose oder auch ein System zur dynamischen Stimulation der Aberrometrie eingesetzt werden. Die geometrischen Daten eines optischen Elements können auch durch Einrichtungen erfasst werden, die auf Basis der optischen Kohärenz Tomografie (OCT) arbeiten oder rotierende Spalt-Scheimpflugkameras, konfokale Laserscanner sowie Ultraschallgeräte umfassen.

Besonders bevorzugt wird zusätzlich zur optischen Analyse der inneren Struktur eine optische Analyse im Bezug auf den aktuellen Alterungszustands des optischen Elements bzw. Linse ermittelt. Auf diese Weise kann für die Linse dann im folgenden Schritt ein individuell angepasstes Therapiemuster mit Hilfe beispielsweise gescannter fs-Laserstrahlung appliziert werden. Gegenüber einer standardisierten Behandlungsform ergibt sich damit der Vorteil, dass eine größtmögliche Akkomodationsbreite bei einem optischen Element, beispielsweise einem gealterten humanen Auge, mit geringstem Aufwand an Schnittfläche oder Thearapieaufwand möglich wird und so übermäßiger Therapieaufwand ausgeschlossen wird. Hierzu wird beispielsweise mit einer dynamischen Wellenfrontdiagnose die dynamischen optischen Eigenschaften des individuellen Auges mit einem dynamischen Aberrometer oder Refraktmeter in Form der jeweiligen Zernikepolynome erfasst. Im einfachsten Fall wird bevorzugt die Wellenfront bei Nahakkomodation und bei Fernakkomodation und die damit verbundene Akkomodationsbreite erfasst. Weitere Eigenschaften, die ermittelt werden können, sind die Geschwindigkeit mit der die Akkomodationsbreite überstrichen wird bzw. die Wellenfrontdaten während dieses Vorgangs. Besonders bevorzugt werden ebenfalls Fehlsichtigkeiten des Patienten, die Hornhauttopographie, die jeweilige Pupillengröße und der Beleuchtungsparameter des Stimulationstargets erfasst. Auf diese Weise erzielt man Informationen über Akkomodationsbreite und Reaktionszeiten des Auges. Hierdurch ist eine umfassende objektive Bewertung der dynamischen Refraktionseigenschaften des Auges verfügbar, womit beispielsweise die Presbyopiestufe charakterisiert werden kann. Im Folgenden kann dann auch diese Information für ein individuelles Schnittmuster, das beispielsweise in einer Steuereinrichtung später errechnet wird, über die Bearbeitungseinrichtung appliziert werden. Es kann so der Akkomodationsprozess ebenfalls in die Finite-Elemente-Simulation einfließen. Durch den radialen Versatz am Ziliarkörper der einzelnen Augen bzw. Linsenbilder im akkomodierten und disakkomodierten Zustand können dann individuell Schnitte bestimmt werden, die bei dieser speziellen Linse zu einer verbesserten Akkomodationsfähigkeit führt. Integriert man in dieser Modellierung dann die fs-Laser-Schnitte kann man resultierende Deformationen feststellen, die mit der verringerten Gewebesteifigkeit korrelieren. Es kann auf diese Weise mit möglichst wenig Schnitten insbesondere außerhalb der natürlichen Tages-Pupillenöffnung im individuellen optomechanischen Augenmodell die größtmögliche Akkomodationsbreite wieder eingestellt werden.

Besonders bevorzugt ist die Apertur, insbesondere die numerische Apertur, der Detektionseinrichtung kleiner als 0,25. Mit einer solchen Apertur ist es möglich, innerhalb eines gealterten humanen Auges die Linse zu untersuchen und zu bearbeiten.

Mit der Apertur ist es möglich, einen Laserfokus mit einem Durchmesser von ca. 5 *µ*m innerhalb der gescannten Linse des humanen Auges bei geweiteter Pupille zu erzeugen, um scannend die Linse zu navigieren und zu therapieren. Entsprechend kann bei einer konfokalen Anordnung der Öffnungsdurchmesser der konfokalen Blende in der Detektoreinheit gewählt werden.

Die Bearbeitungseinrichtung ist eine Komponente, mit der das optische Element modifiziert bzw. bearbeitet werden kann. Dies erfolgt bevorzugt im Inneren des optischen Elements, besonders bevorzugt unter Verwendung von Strahlung. Durch die Bearbeitungseinrichtung ist dann die Struktur innerhalb des optischen Elements veränderbar. Durch diese Einrichtung können Schnitte, Blasen, Blasenfelder, subdisruptive oder disruptive Bereiche innerhalb des optischen Elements kreiert werden. Besonders bevorzugt wird als Bearbeitungseinrichtung in Abhängigkeit von dem zu bearbeitenden Material ein Laser eingesetzt, der so konfiguriert werden kann, dass er durch den Strahlengang in das optische Element eindringen und dort an einer vorbestimmten Stelle im Inneren seine Wirkung erzielen kann. Beim menschlichen Auge wird besonders bevorzugt ein Lasersystem mit Pulsungen im Femtosekunden Bereich (< 1 ps) und einer Wellenlänge im sichtbaren oder NIR-Bereich eingesetzt. Hierüber kann durch die Fokusierung auf die gewünschte Tiefe und die fehlende Absorption der Strahlung durch große Teile des Auges eine Wirkung im Inneren des Auges erzielt werden. Besonders bevorzugt umfasst die Bearbeitungseinrichtung auch Scanner, die einen fokusierten Strahl dreidimensional ablenken und ausrichten.

Bevorzugt wird die Bearbeitungseinrichtung entlang oder im festen Bezug zu der analysierten inneren Struktur des optischen Elements geführt. Hierdurch kann über die Bearbeitungseinrichtung eine Wirkung entlang der inneren Struktur beispielsweise der zwiebelschalenartigen Grenzfläche zwischen den einzelnen Schichten erzeugt werden. Entlang der Struktur bedeutet hierbei, dass sich die durch die Bearbeitungseinrichtung realisierte Wirkung im optischen Element räumlich an den detektieren inneren Strukturen und Schichten orientiert. So ist es beispielsweise denkbar, dass die Bearbeitungseinrichtung eine Wirkung, beispielsweise Schnittmuster, parallel zu der detektierten Grenzfläche in einem vorbestimmten Abstand vornimmt. Es kann auch ein fester Bezug zu der analysierten inneren Struktur und dem Wirkungsbereich der Bearbeitungseinrichtung gewählt sein, so dass beispielsweise die Grenzfläche gewichtet nach der optischen Achse des optischen Elements gemäß einer festzulegenden Funktion berücksichtigt, so dass eine Schnittgeometrie vorgesehen wird, die beispielsweise im Zentrum des optischen Elements einen anderen Abstand zu der detektierten Grenzfläche bzw. inneren Struktur aufweist, als dies im Randbereich der Fall ist. Genauso ist denkbar, dass die Schnittfläche in Sinusform beabstandet zur detektierten Grenzfläche geführt wird. Auf diese Weise wird zwischen der Wirkung der durch die Bearbeitungseinrichtung vorgenommen Modifikation bzw. Bearbeitungen einerseits und der aufgefundenen inneren Strukturen andererseits eine Beziehung einstellbar, die die aufgefundenen Gegebenheiten mit berücksichtigt.

Besonders bevorzugt ist ebenfalls eine Steuereinrichtung vorgesehen, die aus den Daten bzw. Informationen, die über die Detektionseinrichtung gewonnen werden, eine Berechnung der Grunddaten für die Bearbeitungseinrichtung ableitet. Eine solche Berechnung kann beispielsweise auf der Finite-Elemente-Methode beruhen und es werden insoweit entsprechende Daten bestimmt, die für die Koordinaten stehen, aufgrund derer die Bearbeitungseinrichtung dann innerhalb des optischen Elements Modifikationen bzw. Bearbeitungen vornimmt. Es kann dann beispielsweise ein optimiertes Schnittmuster vorgegeben werden, durch das in einem humanen Auge zwischen den einzelnen Grenzschichten bzw. im Bezug auf diese ein Muster bestimmt wird, das die Akkomodationsfähigkeit des Auges bzw. optischen Elements durch Erhöhung der Flexibilität des optischen Elements im Bereich der detektierten inneren Strukturen erhöht. Auf diese Weise ist es möglich, ein gealtertes humanes Auge, das an Presbyopie leidet, wieder mehr Akkomodationsfähigkeit zu verleihen.

Bei einem bevorzugten Ausführungsbeispiel der Erfindung weist die Detektionseinrichtung (10) einen konfokalen Detektor und/ oder einen Detektor auf Basis der Optischen Kohärenztomographie auf.

Besonders bevorzugt wird ein konfokaler Detektor für die Bestimmung der geometrischen Daten des optischen Elements eingesetzt. Ein konfokaler Detektor arbeitet nach dem Prinzip der konfokalen Detektion, die aus der Mikroskopie von teilweise transparentem Gewebe her bekannt ist. Es beruht darauf, dass ein Fokuspunkt eines Lasers durch das Gewebe scannt und das aus diesem Fokuspunkt zurückgestreute Licht durch einen zum Fokuspunkt in einer optisch konjugierten Ebene angeordneten Blende mit einem Detektor registriert wird. Dadurch wird rückgestreutes Licht aus der Umgebung diskriminiert und man erzielt einen hohen Abbildungsfaktor. Während in einem normalen Lichtmikroskop das Bild eine Überlagerung aus einer scharfen Abbildung der Punkte in der Fokalebene und einer unscharfen Abbildung der Punkte außerhalb dieser darstellt, ist bei einem konfokalen Mikroskop das Anregungslicht in die Probe hinein fokusiert. Durch die Abbildung von Licht aus diesem Fokus durch das gleiche Objektiv auf eine Lochblende gelangt es von dort auf einen Detektor (meist ein Photomultiplier oder eine APD).

Anregungs- und Detektionsfokus liegen damit konfokal, also übereinander.
Weiterhin bevorzugt wird ein Dektektor auf Basis der Optischen Kohärenz Tomografie angewandt. Diese Methode wird als OCT abgekürzt (optical coherence tomography) und ist ein Untersuchungsverfahren, bei dem kurzkohärentes Licht mit Hilfe eines Interferometers zur Entfernungsmessung reflektiver Materialien eingesetzt wird. Das entsprechende akustische Verfahren ist die Ultraschalldiagnostik (Sonografie). Hierüber lassen sich aufgrund der Reflexionen an Grenzflächen von Materialien mit unterschiedlichen Brechzahlen ein dreidimensionales Bild des optischen Elements rekonstruieren. Diese Rekonstruktion wird als Tomografie bezeichnet. Im Stand der Technik wird diese Untersuchung eingesetzt, um beispielsweise den Augenhintergrund bzw. den hinteren Augenabschnitt zu untersuchen, da konkurrierende Technologien wie z.B. das Konfokalmikroskop sich durch eine Schichtstruktur der ca. 250 bis 200 *µ*m dicken Netzhaut aufgrund der geringen Pupillengröße und des großen Abstands von Hornhaut zu Netzhaut nur unzureichend abbilden können. Erfindungsgemäß wird dieses Verfahren nun aber entgegen der bisherigen Übung mit der vorgeschlagenen Navigationsvorrichtung einsetzbar, um die Augenlinse zu untersuchen und zu bearbeiten.

Bei einem Ausführungsbeispiel der Erfindung ist die Apertur der Detektionseinrichtung kleiner 0,25 und größer 0,1, insbesondere kleiner 0,22 und größer 0,18.
Mit einer Apertur der Detektionseinrichtung in dieser Größenordnung ist besonders bevorzugt eine Untersuchung der Linse eines gealterten humanen Auges möglich. Besonders bevorzugt beträgt die Apertur ungefähr 0,2.

Bei einem bevorzugten Ausführungsbeispiel der Erfindung weist die Detektionseinrichtung eine polarisationsoptische Sensorik auf.

Eine polarisationsoptische Sensorik ermöglicht bei Verwendung von polarisiertem Licht die Spannungsverteilung in lichtdurchlässigen Körpern zu untersuchen. Durch Verwendung von monochromatischem Licht entsteht ein System aus dunklen hellen Streifen, deren Anordnung zuverlässige Rückschlüsse auf Verteilung und Größe der mechanischen Spannung an allen Stellen des optischen Körpers erlaubt. Auf diese Weise ist durch die Detektionseinrichtung nochmals zusätzlich die innere Struktur im Hinblick auf die Spannungsverteilung in dem optischen Element untersuchbar.

Bei einem bevorzugten Ausführungsbeispiel der Erfindung weist die Bearbeitungseinrichtung einen Laser, insbesondere einen fs-Laser, insbesondere einen Laser mit einem gescannten, fokussierten, ultrakurz gepulsten Laserstrahl auf.

Als Laser werden bevorzugt gescannte, fokusierte fs-Laserstrahlen einer Pulsbreite von kleiner 1 ps, besonders bevorzugt etwa 300 fs, mit einer Pulsenergie von 0,1 bis 10 *µ*J, vorzugsweise 1 *µ*J und einem Fokusdurchmesser von etwa 5 *µ*m eingesetzt. Die Wellenlänge des Lasersystems ist bevorzugt zwischen 400 und 1300 nm, besonders bevorzugt zwischen 780 und 1060 nm. Der fs-Laser ist ein Laser, der hoch intensive Lichtimpulse aussendet, deren Dauer im fs-Bereich liegt. Fs-Laser komprimieren die Energie über Modenkopplung in einen extrem kurzen Zeitbereich und haben deshalb gegenüber Dauer-Lasern wesentlich höhere Spitzenleistungen. Der Vorteil der Anwendung von fs-Lasern besteht insbesondere in hohen Spitzenintensität, der geringen Wärmeübertragung auf das Substrat und dem breitbandigen Spektrum. Der fs-Laser ist in der Messtechnik ebenfalls im Bereich der Optischen Kohärenz Tomografie einsetzbar. Daher ist es besonders bevorzugt möglich, einen fs-Laser für die Detektionseinrichtung im Rahmen einer Optischen Kohärenz Tomografie einzusetzen und mit dem selben fs-Laser als Element der Bearbeitungseinrichtung die Modifikationen und Bearbeitungen im optischen Element vorzunehmen. Die Detektionseinrichtung und die Bearbeitungseinrichtung fallen dann konstruktionstechnisch weitgehend zusammen, da sie auf den selben fs-Laser zurückgreifen.

Bei einem bevorzugten Ausführungsbeispiel der Erfindung ist die Bearbeitungseinrichtung so ausgelegt, um Strahlung im optischen Element zu erzeugen, die subdisruptiv mit Pulsenergien kleiner gleich 1 mikroJ stimuliert und/ oder disruptiv mit Pulsenergien größer 0,1 mikroJ perforiert und/ oder durch enge Aneinanderreihung der durch Disruption erzeugten Blasen zu schneiden. Auf diese Weise ist es möglich, durch die Bearbeitungseinrichtung das optische Element in unterschiedlicher Art und Weise zu modifizieren bzw. zu bearbeiten. Von leichten Stimulierungen bis hin zum Anbringen durchgehender Schnitte ist auf diese Weise jegliche Abstufung möglich.

Beim Anwenden von Ultrakurzpuls-Laser Strahlung kommt es aufgrund der fokusierten Strahlen je nach Energiewahl zu subdisruptiver oder disruptiver Modifikation im Zielgebiet des Strahles im optischen Element. Eine Disruption ist eine mechanische Verformung, die aufgrund der Verdampfung von Material im Zielgebiet und der damit verbundenen Ausdehnung mechanisch realisiert wird. Bei einer subdisruptiven Stimulation des Zielgebietes werden Pulsenergien geringer Intensität eingesetzt, so dass dieser mechanische Effekt, wie er beispielsweise auch beim Ultraschall auftritt, nicht vorkommt.

Wenn höhere Pulsenergien gewählt werden, kommt es zum disruptiven mechanischen Verändern des Zielgebiets durch eine entsprechende Blasenbildung innerhalb des Gewebes. Der Spotdurchmesser der einzelnen Bläschen beträgt hierbei bevorzugt wenige *µ*m. Auf dieser Weise ist es möglich, das Zielgebiet zu perforieren, d.h. bleibende Löcher zu bilden. Beim Auge werden diese Löcher dann durch Flüssigkeit bzw. benachbartes flüssigeres Gewebe schnell aufgefüllt. Im Endefekt wird durch diese eingebrachte Struktur und Modifikation im optischen Element die Verformbarkeit des optischen Elements erhöht, da durch die Wegnahme des entsprechenden Materials an den beschossenen Stellen das gesamtoptische Element flexibler und besser verformbar wird. Besonders bevorzugt ist es auch möglich, durch Aneinanderreihung von einzelnen disruptiv generierten Blasen mit einem Spotdurchmesser und Spotabstand von wenigen *µ*m diese so vollständig zu überlagern, dass ein durchgehender Schnitt entsteht. Diese Schnittgeometrien und Strukturen können dann in das Auge bzw. optische Element eingebracht werden und verlaufen besonders bevorzugt entlang der detektierten geometrischen inneren Struktur des optischen Elements. Durch diese Blasenfelder kann ein Ultrakurzpuls-Lasersystem Strukturen erzeugen, die beispielsweise im jeweils senkrechten Abstand von 0,2 mm von einer der detektierten Grenzschichten geführte Schnitte realisiert. Genauso ist es denkbar, dass mit Bezug auf diese detektierte Grenzschicht im Inneren des optischen Elements mit einer sinusförmigen Modulation von 0,1 und 1 mm Abstand geführte Schnitte umgesetzt werden. Weiterhin sind unter diesen Strukturen beliebige geometrische Schnittmuster oder Blasenfelder zu verstehen, die innerhalb der Linse des optischen Elements mit einer vorher mit Hilfe des Messsystems ausgemachten anatomischen Struktur in eine feste geometrische Beziehung gesetzt werden.

Bei einem bevorzugten Ausführungsbeispiel der Erfindung umfasst das optische Element (50) eine Linse, insbesondere eine gealterte Augenlinse.

Eine gealterte Augenlinse weist oftmals Verhärtungen auf, die zu einem Verlust der Akkomodationsfähigkeit des Auges führen.

Das Auge hat dabei aufgrund der Wachstumsphasen verschieden Kerne, die sich vom Alter her unterscheiden und zwiebelartig übereinander angeordnet sind. Bei dem innersten und damit ältesten Kern kommt es im Alter zu Verhärtungen. Mit der vorliegenden Erfindung kann nun eine Vorrichtung bereit gestellt werden, die die entsprechenden Grenzstrukturen zwischen den einzelnen Kernschichten und Bestandteilen detektiert, um dann den verhärteten älteren im Inneren gelegenen Kern durch Anbringen entsprechender Schnittmuster bzw. Bläschenstrukturen wieder mehr Flexibilität zu verleihen. Es ist auch denkbar, einen oder mehrere dieser inneren verhärteten Kerne komplett zu entnehmen und durch Gel auszutauschen.

Bei einem bevorzugten Ausführungsbeispiel der Erfindung umfasst die Navigationsvorrichtung (1) eine ophtalmologische Saug-Spüleinrichtung (30) mit mindestens einer Kanüle (35).

Besonders bevorzugt wird mit einer solchen Vorrichtung ein innerer Kern der menschlichen Augenlinsen, der erhärtet ist, zerkleinert, entfernt und durch ein entsprechendes Gel ersetzt. Auf diese Weise ist es möglich, lediglich den innersten Kern der Linse auszutauschen und durch die äußersten Schichten des Kerns der Linse weiterhin ein tragfähiges Gerüst beizubehalten, das auch dafür sorgt, dass das eingebrachte Gel am Platz des vormals inneren Kern verbleibt. Dadurch kann die Flexibilität der Augenlinse und damit die Akkomodationsfähigkeit stark verbessert werden.

Besonders bevorzugt wird hierfür eine ophtalmologische Saug-Spüleinrichtung verwendet, wie sie aus der Phakolmulsifikation bekannt ist. Diese weist typischerweise ein bis zwei Kanülen auf, über die dann der zerkleinerte Kern abgesaugt werden kann. So ist es möglich, mit einem fs-Laser den inneren Kern einer Augenlinse in kleine, über eine Saug-Spüleinheit absaugbare Teil von einem Durchmesser von weniger als einem mm zu zerlegen. Dieser präparierte Linsenkern wird mit Hilfe einer Saug-Spüleinheit wie sie beispielsweise zur konventionellen Phakoemulsifikationstechnik her bekannt ist oder mit einem Gerät mit anderen Saug-Spülprinzip vollständig ausgeräumt. Der entstandene hohle Linsenkörper kann nun bevorzugt über eine Kanüle oder vorzugsweise bimanuell, d.h. mit Hilfe von zwei gegenüberliegenden eingestochenen Kanülen, mit einem künstlichen oder aus der Natur gewonnenen biokompatiblen, flexiblen, transparenten Gelmaterial gefüllt werden, um die optische Funktion der Linse und ihre Akkomodationsfähigkeit wieder herzustellen.

Besonders bevorzugt weist die Kanüle eine Spitze auf, die so gestaltet ist, dass beim Einstechen insbesondere in die Linsenrinde das Material zur Seite gedrängt und nicht nach vorne geschoben wird, so dass nach dem Befüllen mit Gel ein Selbstdichtungseffekt entsteht. Mit dieser Kurzpuls-Laser gestützten partiellen Phakoemulsifikation vermeidet man die Entstehung eines Nachstars und umgeht zudem weiterhin einen Mangel bei aktuell in klinischer Untersuchung befindlicher Gelfüllungen des Kapselsacks nach vorheriger vollständiger Phakoemulsifikation. Das Problem ist hierbei, dass der gesamte Linsenkern ausgeräumt werden muss und das Gel dadurch nicht gut fixiert werden kann. Außerdem können durch wuchernde restliche Zellen der entfernten Linse der hinteren Kapselsackmembran eine Eintrübung erzeugt werden. Dieser Nachstar (nachträgliche Eintrübung) wird konventionell durch Photodisruption mit Hilfe eines gütegeschalteten Nd: YAG-Lasers behandelt, wodurch die eingetrübte hintere Kapselsackmembran aus dem optischen Strahlengang des Auges entfernt wird. Bei vollständiger Gelfüllung des Kapselsacks ist jedoch wegen des dann auslaufenden Gels diese Methode dann nicht mehr möglich. Durch die erfindungsgemäße partielle Gelfüllung innerhalb der Rinde der natürlichen Linse ist einerseits die Nachstarrate stark reduziert und andererseits das Gel nochmals gekapselt, sowie insbesondere der harte Kern mit einem flexiblen Gel ausgetauscht, um die Akkomodationsfähgikeit wieder herzustellen.

In einer weiteren vorteilhaften Ausführungsform wird nach Befüllung des durch die teilweise Phakoemulsifikation entstandenen Linsenkörper das eingebrachte Füllmaterial mit elektromagnetischer Strahlung, z.B. UV-Strahlung, bestrahlt, was zu einer Veränderung der Konsistenz bzw. Viskosität des Gels führt. Dadurch werden Leckagen bzw. das nachträgliche Austreten von Gel aus dem Linsenkörper vermieden.

In einer weiteren vorteilhaften Ausführungsform kann über die Bestrahlung mit elektromagnetischer Strahlung, z.B. UV-Strahlung, nachträglich eine Feinabstimmung der Brechkraft des neuen gelartigen Linsenkerns mittels objektiver und/oder subjektiver Bewertung durch den Patienten erfolgen.

Eine weitere Anwendung findet die erfindungsgemäße Vorrichtung des intraokularen navigierten Ultrakurzpuls-Laserschneidens zur Ultrakurzpuls-Laser-Fragmentierung der gesamten Linse für die herkömmliche Phakoemulsifikation der vollständigen Linse. Dabei kann man auf die konventionelle Verflüssigung der Linse mit Hilfe von Ultraschall verzichten und die durch die beschriebenen Laserschnitte zerkleinerte Linse lediglich mit der Saug-Spül-Einheit absaugen. Durch diese neue Methode wird die invasive OP Zeit und die thermische Last am Auge reduziert.

Weiterhin findet die erfindungsgemäße Vorrichtung des intraokularen navigierten Ultrakurzpuls-Laserschneidens eine Anwendung zum Schneiden der hinteren Kapselsackmembran zur Behandlung der Nachstars. Gegenüber dem konventionellen ns Nd:YAG-Laser ergibt sich mit dem fs-Laser eine schonendere Behandlung aufgrund der geringeren Disruptionsenergien, die sich von einigen mJ auf wenige *µ*J reduzieren. Während bei konventionellen Nd:YAG-Disruptionslasern der Fokus hinter die Kapselsackmembran gelegt wird, um mit der akustischen Schockwelle die Membran zu zerstören und die Kunstlinse zu schützen, wird mit Hilfe des hochrepetierenden fs-Lasers und der gekoppelten Navigation die Fokussierung auf oder in unmittelbarerer Nähe der Membran erfolgen.

Die Aufgabe wird auch gelöst durch eine Therapieplanungs-Vorrichtung zur Planung der Therapie eines humanen Auges umfassend ein dynamisches Wellenfrontmeßgerät (Aberrometer) zur Ermittlung von Wellenfrontdaten und ein Diagnostik Gerät zur Bestimmung von geometrischen Parametern des Optikapparates des Auges (Vorderkammer OCT und/oder rotierende Scheimpflugkamera und/oder Ultraschall-3d Meßsystem und oder Augenlängenmeßgeräte und oder Topografiemeßgerät) und eine Steuereinrichtung zur konsistenten Überlagerung der ermittelten Wellenfrontdaten und der Geometriedaten und eine weitere Steuereinrichtung zur Planung der effizientesten therapeutischen Laserschnittführung.

Als dynamisches Wellenfrontmessgerät kann ein Aberrometer verwendet werden. Hierüber werden Wellenfrontdaten des gesamten Auges ermittelt.

Als Diagnostik Gerät zur Bestimmung von geometrischen Parametern des Optikapparates des Auges kann ein Vorderkammer OCT und/oder eine rotierende Scheimpflugkamera und/oder ein Ultraschall-3d Messsystem und oder Augenlängenmessgeräte und/ oder ein Topografiemessgerät eingesetzt werden. Hierüber werden Daten vor allem der Linse ermittelt.

Mit der Steuereinrichtung wird ein erstes Softwarepaket genutzt, um eine konsistenten Überlagerung der ermittelten Wellenfrontdaten und der Geometriedaten zu erreichen. Hierdurch wird ein Gesamtbild der Linse und dem Gesamtoptischen Apparat des Auges gegeben.

Mit einer weiteren Steuereinrichtung - bevorzugt wird hierfür auch dieselbe Steuereinrichtung wie im vorhergehenden Schritt genutzt - wird mit einem zweiten Softwarepaket die Planung der effizientesten therapeutischen Laserschnittführung vorgenommen. Hierbei kann nicht nur die Geometrie der Linse berücksichtigt werden, sondern auch etwaige Fehlsichtigkeiten des Gesamtapparates und so, beispielsweise über die Finite-Elemente-Methode, eine Schnittführung ermittelt werden, die die individuellen Gegebenheiten dieses Auges berücksichtigt und hier zu einer Verbesserung der Akkomodationsfähigkeit führt.

Die Aufgabe wird auch gelöst durch ein Verfahren zur Modifikation eines optischen Elements, insbesondere einer humanen Augenlinse mit Hilfe eines bevorzugt gescannten, fokusierten und gepulsten Laserstrahls, wobei die Modifikation nach den folgenden Schritten erfolgt:
vollständige Erfassung der dynamischen optischen Wellenfront des Auges während der Akkommodation und der zugehörigen geometrischen Parameter zur individuellen Planung einer fs-Laser-Schnitttherapie,
Detektion von Position, Geometrie und innerer Struktur der Augenlinse,
Auswahl eines aus mehreren Flächen bestehenden Grundmusters für die Schnittführung,
Erstellung eines Schnittführungsprotokolls durch Anpassung bzw. Umrechnung des Grundmusters an die detektierte individuelle Geometrie der Augenlinse,
Indifferenzierung des Positionierungssystems zu der Position der Augenlinse und
Durchführung der chirurgischen Schnitte mit dem Lasersystem nach dem erstellten Protokoll.

Die Erfindung soll nun anhand von Zeichnungen näher erläutert werden. Hier sind in den Figuren vorteilhafte Ausführungsbeispiele dargestellt. Es zeigen:
- Figur 1: eine schematische Darstelltung einer Navigationsvorrichtung gemäß der vorliegenden Erfindung;
- Figur 2: eine schematische Ansicht des Aufbaus der menschlichen Augenlinse;
- Figur 3: eine schematische Ansicht zweier Schnittführung im optischen Element und
- Figur 4: eine schematische Ansicht einer weiteren Ausführungsform der vorliegenden Erfindung bei Anwendung in der partiellen Phakoemulsifikation.

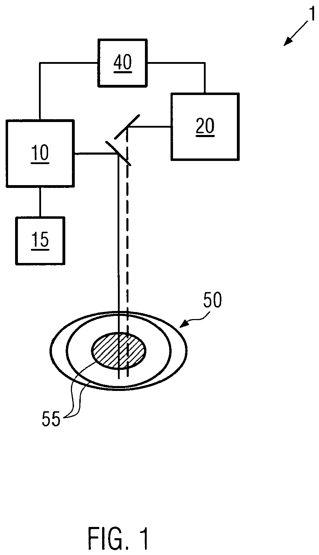

In **Figur 1** ist eine schematische Darstellung einer Navigationsvorrichtung 1 gemäß der vorliegenden Erfindung dargestellt. Die Navigationsvorrichtung 1 umfasst eine Detektionseinrichtung 10, die mit einer optischen konfokalen und/oder optischen Kohärenz Tomografieeinrichtung sowie mit einer polarisationsoptischen Sensorik 15 ausgestattet ist. Daneben ist eine Bearbeitungseinrichtung 20 vorgesehen. Die Detektionseinrichtung 10 sowie die Bearbeitungseinrichtung 20 sind mit einer Steuereinrichtung 40 verbunden. Ein entsprechender optischer Pfad kann von der Detektionseinrichtung 10 sowie von der Bearbeitungseinrichtung 20 über Scannerspiegel in ein optisches Element 50, hier eine mehrschichtige Linse, gerichtet werden. In der Linse 50 sind verschiedene innere Strukturen des optischen Elements mit dem Bezugszeichen 55 bezeichnet.

Über die Detektionseinrichtung 10 wird die innere Struktur 55 der Linse 50 detektiert. Hierbei unterstützt eine Sensorik 15 und vermag so nochmals ein umfangreicheres und auch vom Spannungsverhältnis abhängendes dreidimensionales Bild dieser inneren Struktur 55 zu ermitteln. Die Detektionseinrichtung 10 umfasst bevorzugt zusätzlich auch eine Einrichtung zur dynamischen Wellenfrontdiagnose, um bei Nahakkomodation und bei Fernakkomodation die Akkomodationsbreite des optischen Elements bzw. der Augenlinse zu erfassen. Ebenso wird damit die Geschwindigkeit bestimmt, mit der die Akkomodationsbreite überstrichen wird. Daneben werden bevorzugt durch die Detektionseinrichtung 10 ebenfalls die Fehlsichtigkeit die Hornhauttopographie sowie die jeweilige Pupillengröße und Beleuchtungsparameter des Stimulationstargets erfasst. Besonders bevorzugt werden selektiv Sphäre, Zylinder oder beliebige höhere Aberrationen dynamisch analysiert. Außerdem kann die Detektionseinrichtung 10 auch geometrische Daten des Auges erfassen wie beispielsweise mit Geräten auf Basis der optischen Kohärenz Tomografie oder rotierenden Spalt-Scheimpflugkameras, konfokalen Laserscanern sowie mit Ultraschallgeräten. Diese Informationen werden an die Steuereinrichtung 40 übergeben, in der über ein Finite-Elemente-Modell Schusskoordinaten für die Bearbeitungseinrichtung 20 berechnet werden. Besonders bevorzugt werden die Daten zuerst an die Steuereinrichtung übergeben, um hier bevorzugte Schnittgeometrien zu berechnen, die bei Applikation in das Auge zu einer Erhöhung beispielsweise der Akkomodationsfähigkeit führen. So kann mit Hilfe beispielsweise der finiten Elementemethode ein Muster bestimmt werden, das den höchsten Akkomodationshubzuwachs verspricht. Nachdem die Simulation abgeschlossen ist werden somit Schussparameter bereit gestellt, um dann über den Laser diese Schnittgeometrien in das optische Element bzw. in die Augenlinse zu aplizieren. Die Steuereinrichtung gibt diese Daten an die Bearbeitungseinrichtung 20 weiter und von dort wird die entsprechend vorbestimmte Bearbeitung der Linse 50 vorgenommen. Hierbei orientiert sich das Schussmuster und die Schnittgeometrien, die nun aufgebracht werden, an der inneren Struktur 55 der Linse 50. Es können so therapeutische Schnitte durchgeführt werden, die entlang der detektierten Flächen oder diesen Flächen zugeordneten geometrischen Strukturen verlaufen und beispielsweise mit Hilfe von Blasenfeldern eines Ultrakurzpuls-Lasersystem als Bearbeitungseinrichtung 20 erzeugt werden. Die Erfindung bietet damit den Vorteil eines diagnostisch und therapeutisch gekoppelten Systems zu zweckmäßigen und minimalinvasiven Therapie unter bestmöglicher Anpassung an die individuelle Anatomie einer menschlichen Augenlinse. Durch die anatomisch gekoppelte Schnittführung werden natürliche Gleitebenen regeneriert und damit eine hohe physiologische Verträglichkeit der Therapie gesichert.

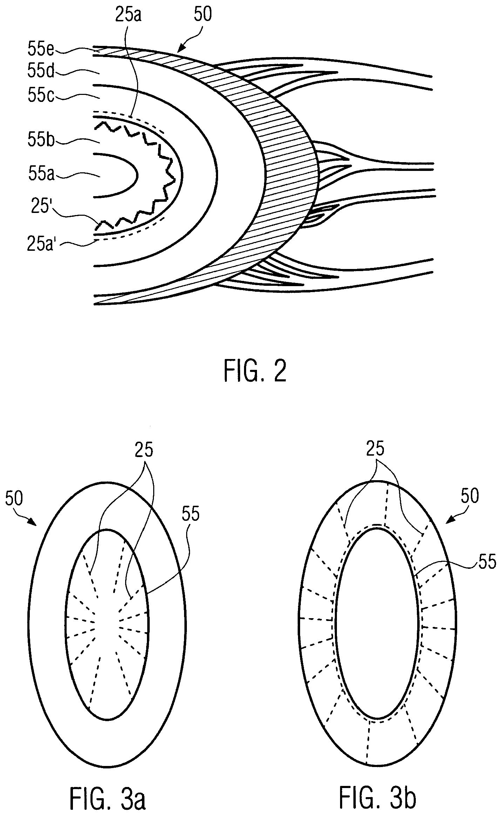

In **Figur 2** ist eine schematische Ansicht des Aufbaus des menschlichen Auges dargestellt. Zusätzlich dargestellt sind Schnittmuster, die mit der erfindungsgemäßen Vorrichtung gemäß Figur 1 eingebracht worden sind.

Als optisches Element 50 ist die Struktur eines humanen Auges mit dem Bezugszeichen 50 bezeichnet. Das Auge hat eine individuelle Geometrie und anatomisch existierende Diskontinuitätsflächen, beispielsweise Grenzschichten zwischen den einzelnen Kernbestandteilen. So ist der Embryonalkern 55a, der Fetalkern 55b, der Adultkern 55c sowie die Rinde 55d dargestellt. Die vordere Kapsel ist mit 55e bezeichnet. Es existieren im Auge 50 damit natürliche Grenzflächen bzw. Grenzschichten, beispielsweise zwischen dem Embryonalkern 55a und dem Fetalkern 55b bzw. Fetalkern 55b und dem Adultkern 55c, etc.. Diese Grenzschichten sind in der Figur mit einer durchgehenden Linie gekennzeichnet. Daneben sind mit punktierten bzw. gestrichelten Linien Schnittmuster 25 eingezeichnet. So existiert ein Schnittmuster 25' entlang der Grenzschicht zwischen Fetalkern 55b und Adultkern 55c auf Seiten des Fetalkerns 55b. Dieses Schnittmuster 25' ist sinusförmig entlang der Grenzschicht bzw. inneren Struktur 55 der Linse 50 ausgeführt. Daneben sind Schnittmuster 25a und 25'a dargestellt, die in der Nähe der Grenzschicht zwischen dem Adultkern 55c und dem Fetalkern 55b auf Seiten des Adultkerns in etwa parallel zu dieser Grenzschicht mit einem vorgegebenen Abstand ausgeführt worden sind.

Durch die Einbringung der Schnittmuster 25, die entlang der detektierten Flächen oder diesen Flächen zugeordneten geometrischen Strukturen verlaufen und mit Hilfe von Blasenfeldern eines Ultrakurzpuls-Lasersystems erzeugt werden können, wirken diese als anatomisch gekoppelte Schnittführung und erhöhen die Flexibilität der Schicht zwischen dem Fetalkern 55b und dem Adultkern 55c. Auf diese Weise kann das Auge nun besser akkomodiert werden und verliert insoweit teilweise die Einschränkungen aufgrund einer altersbedingten Verhärtung des Linsenkerns. Besonders bevorzugt wird die Schnittführung der Schnittmuster 25 vom zum Apex der Kornea am weitesten entfernten Punkt im Inneren des Auge begonnen, um die bestmöglichste Fokusqualität des Laserspots zu sichern und sukzessive Streuzentren für die parasitäre Laserstrahlung der folgenden Laserspots zu erzeugen. Auf diese Weise kann die retinale Laserbelastung minimiert werden.

In **Figur 3** ist eine schematische Ansicht zweier Schnittführungen im optischen Element dargestellt. Die Figur zerfällt in zwei Teilfiguren Figur 3a und Figur 3b. Es sind in den beiden Figuren unterschiedliche Schnittmuster aufgeführt.

In Figur 3a ist sehr schematisch und vereinfacht eine Linse 50 dargestellt, die eine innere Struktur 55 in Form eines verhärteten inneren Kerns aufweist. Mit gestrichelten Linien ist dann eine erste mögliche Schnittmustergeometrie 25 dargestellt, die der Linse 50 eine erhöhte Flexibilität und damit Akkomodationsfähigkeit verleihen soll. Die Schnitte des Schnittmusters 25 sind radial vom Zentrum des Linsenkerns nach außen gerichtet und enden an den geometrischen Diskontinuitätsflächen 55 der Linse. Aufgrund der zylindrischen Geometrie der Linse erfolgen die im Querschnitt dargestellten Schnitte auf Kegelsegmenten. In der Abbildung Figur 3a verlaufen die Schnitte nur im Linsenkern, da dort die Festigkeit am höchsten ist und somit ein maximaler Effekt zur Wiederherstellung der Akkomodationsfähigkeit erzielt werden kann.

In Abbildung Figur 3b wird ein Muster 25 dargestellt, das besonders günstig für Patienten ist, bei denen zum Beispiel mit einer Kataraktbildung im Kern gerechnet werden muss. Die radiale Schnittführung erfolgt nur im Kortex, wobei zusätzlich zu den radialen Schnitten entlang der Diskontinuitätsfläche zwischen Kern und Kortex geschnitten wird. Bevorzugt wird dabei das Schnittmuster in bekannter Weise mit einem gescannten, fokusierten fs-Laserstrahl einer Pulsbreite kleiner 1 ps (vorzugsweise 300 fs) mit einer Pulsenergie von 0,1 bis 10 *µ*J, vorzugsweise 1 *µ*J und einem einem Fokusdurchmesser von ca. 5 *µ*m vorgenommen. Die Wellenlänge des Lasersystems liegt bevorzugt im Bereich 400 bis 1300 nm, besonders bevorzugt zwischen 780 und 1060 nm.

Bevorzugt kann die anatomisch gekoppelte Schnittführung nicht über den gesamten Linsendurchmesser durchgeführt werden, sondern nur in einem Randbereich, wobei eine optische Zone des Auges mit z.B. 3 mm Durchmesser, bevorzugt 2 bis 7 mm Durchmesser unbehandelt bleibt oder nur in einem zentralen Pupillenbereich von z.B. 7 mm Durchmesser behandelt wird. Dabei bleibt ein Randbereich unbehandelt. Bevorzugt werden bei einer Behandlung am Randbereich vorzugsweise Spiegelkontaktgläser und bei einer Behandlung im zentralen Bereich einfache Kontaktgläser eingesetzt.

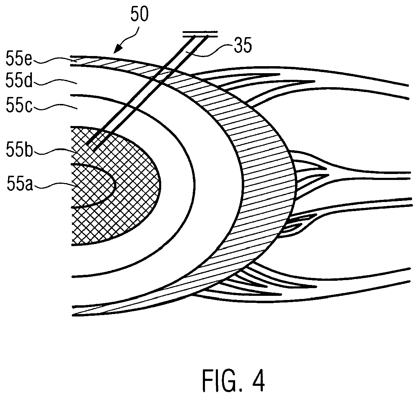

In **Figur 4** ist eine schematische Ansicht einer weiteren Ausführungsform der vorliegenden Erfindung bei Anwendung in der partiellen Phakoemulsifikation dargestellt. Wie in Figur 2 zeigt auch Figur 4 einen schematischen Querschnitt durch das humane Auge mit den verschiedenen Kernen und entsprechenden Grenzflächen. In Figur 4 ist zu erkennen, dass der Embryonalkern 55a und der Fetalkern 55b hier gekreuztgestrichelt dargestellt emulsifiziert wurden, d.h., dass die Kerne in kleinste Teile mit einem Durchmesser von weniger als einem Millimeter zerkleinert wurden. Es ist außerdem eine Kanüle 35 dargestellt, die seitlich in die Linse 50 eingebracht ist und über die dann einerseits das zerkleinerte Material abgesaugt werden kann als auch eine entsprechende Gelfüllung anschließend eingebracht werden kann. Es ist zu beachten, dass besonders bevorzugt zwei Kanülen 35 eingesetzt werden, wobei die zweite Kanüle von der anderen Seite in die Linse eingebracht wird, was hier durch die hälftige Darstellung der Linse nicht eingezeichnet ist. Auf diese Weise kann über die eine Kanüle Material abgesaugt werden, während über die andere Kanüle Spülmittel eingebracht wird und so den Absaugvorgang unterstützen bzw. verbessern kann. Genauso ist es später dann möglich, das Gel einzubringen und das noch vorhandene Spülmittel, das durch das Gel verdrängt wird, über die entsprechende saugende Kanüle abzuführen. Idealerweise wird über eine Kanüle damit ständig Material zugeführt und über die andere Kanüle abgesaugt.

Auf diese Weise wird der innere harte Kern - in diesem Fall bestehend aus den beiden Segmenten des Embryonalkerns 55a und Fetalkerns 55b (es könnte sich auch lediglich um einen Kern handeln) der gealterten menschlichen Augenlinse durch die gescannten Kurzpulslaser-Spots ausgeschnitten und gleichzeitig in kleine, über eine Saug-Spüleinheit absaugbare Teile von einem Durchmesser von weniger als 1 mm zerlegt. Dieser präparierte Linsenkern wird dann mit Hilfe einer Saug-Spüleinheit wie sie aus der konvenzionellen Phakoemulsifikationstechnik her bekannt ist oder mit einem Gerät mit anderem Saug-Spülprinzip vollständig ausgeräumt. Der entstandene hohle Linsenkörper wird nun erfindungsgemäß über eine Kanüle oder vorzugsweise bimanuell, d.h. mit Hilfe von zwei gegenüberliegenden eingestochenen Kanülen, mit einem künstlichen oder aus der Natur gewonnen, biokompatiblen, flexiblen, transparenten Gelmaterial gefüllt, um die optische Funktion der Linse und deren Akkomodationfähigkeit wieder herzustellen. Bevorzugt werden mit diesen Kanülen, die ca. 1 mm Durchmesser haben, das Auge inklusive Kapselsack und Linsenrinde durchstochen und dabei bei der Präperation möglichst schräge Einstiche vorgenommen, um nach der Behandlung den Selbstdichtungseffekt ausnutzen zu können. Besonders bevorzugt wird zusätzlich die Gelkonsistenz im Zusammenhang mit den Durchmessern der Einstiche so ausgelegt, dass ein Schließen der Öffnungen gegeben ist. Besonders bevorzugt werden die Kanülen an ihrer Spitze so ausgestaltet, dass beim Einstechen insbesondere in die Linsenrinde das Material zur Seite gedrängt und nicht nach vorne geschoben wird, so dass ein Selbstabdichtungseffekt entsteht.

Durch diese bevorzugte auf Kurzpuls-Laser gestützte partielle Phakoemulsifikation vermeidet man die Entstehung eines Nachstars und umgeht zudem weiterhin einen Mangel bei aktuellen klinischer Untersuchung befindlicher Gelfüllungen des Kapselsacks nach vorheriger vollständiger Phakoemulsifikation. Dieser Nachteil besteht darin, dass der zu ca. 50% auftretende Nachstar, bei dem sich durch wuchernde restliche Zellen der entfernten Linse auf der hinteren Kapselsackmembran eine Eintrübung ergibt, konventionell durch Photodisruption mit Hilfe eines gütegeschalteten Nd:YAG-Lasers behandelt wird. Hierdurch wird die eingetrübte hintere Kapselsackmembran aus dem optischen Strahlengang des Auges jedoch entfernt. Eine vollständige Gelfüllung des Kapselsacks ist dann wegen des nun auslaufenden Gels nicht mehr möglich. Durch die erfindungsgemäß vorgestellte partielle Gelfüllung innerhalb der Rinde der natürlichen Linse ist einerseits die Nachstarrate stark reduziert und andererseits das Gel nochmals gekapselt.

Die Schnittführung bei der partiellen Phakoemulsifikation zur partiellen Entfernung der Linse in Form eines inneren Kerns erfolgt dabei entlang des inneren Kerns vollständig, wobei zusätzlich der zu entfernen Kern mittels effizienten geometrischen Schnittmustern fragmentiert wird. Besonders bevorzugt wird dabei vom zum Apex der Kornea am weitesten entfernten Punkt im Inneren des Auges begonnen, um die bestmöglichste Fokusqualität des Laserspots zu sichern und sukzessive Streuzentren für die parasitäre Laserstrahlung der folgenden Laserspots zu erzeugen und dabei die retinale Laserbelastung zu minimieren.

Besonders bevorzugt wird nach Befüllen des durch die teilweise Phakoemulsifikation entstandene Linsenkörpers das eingebrachte Füllmaterial mit elektromagnetischer Strahlung, z.B. UV-Strahlung, bestrahlt, was zu einer Veränderung der Konsistenz bzw. Viskosität des Gels führt. Dadurch können einerseits Lekagen bzw. das nachträgliche Austreten von Gel aus dem Linsenkörper vermieden werden. Andererseits ist es möglich eine nachträgliche Feinabstimmung der Brechkraft des neuen gelartigen Linsenkerns mittels objektiver und/oder subjektiver Bewertung durch den Patienten durchzuführen. Damit kann eine für die dynamische Refraktion effiziente Brechzahlkradienten in der regenerierten Linse hergestellt werden.

Es wird somit ein menschliches Auge genau vorher untersucht, um die Wellenfrontdynamik vollständig zu bestimmen (hat das Auge noch 4, 3, 2 oder 1 dpt. Akkomodationsbreite?). Außerdem wird bevorzugt die zugehörige individuelle geometrische Form zur mindest jeweiligen Nah- und Ferneinstellung (Krümmung der Linsenvorder- und -rück-seite sowie Abstände der optischen Flächen, ...) erfasst. Daraus wird mit Hilfe einer Software, z.B. auf Basis der finiten Elemente Methode dieses individuelle optische/geometrische System vollständig beschrieben. Eine weitere Software kann daraus die optimierten Linsenschnitte berechnen, welche vom Lasersystem auszuführen sind.

Auf diese Weise ist ein ophtalmolochirurgisches Gerätsystem und eine Anleitung zum Umgang mit diesem zur schonenden und präzisen Präperation oder Entfernung von transparenten biologischem Gewebe, insbesondere im Auge und speziell in der menschlichen Augenlinse im Rahmen einer refraktiven Laserchirurgie oder Katarakt-OP insbesondere zur Behandlung der Präsbyopie durch Regeneration der Akkomodationsfähgikeit des Linsenkörpers bereit gestellt worden.

### Bezugszeichenliste

- 1: Navigationsvorrichtung
- 10: Detektionseinrichtung
- 15: polarisationsoptische Sensorik
- 20: Bearbeitungseinrichtung
- 25: Schnittmuster
- 30: ophtalmologische Saug-Spüleinrichtung
- 35: Kanüle
- 40: Steuereinrichtung
- 50: Optisches Element / Linse
- 55: Innere Struktur des Optischen Elements / Linse

## Patentansprüche

1. Navigationsvorrichtung (1) zur optischen Analyse der inneren Struktur (55) eines optischen Elements (50) und zur Bearbeitung des optischen Elements (50) umfassend eine Detektionseinrichtung (10) und eine Bearbeitungseinrichtung (20), die eingerichtet ist, entlang oder im festen Bezug zu der analysierten inneren Struktur (55) des optischen Elements (50) geführt zu werden, **dadurch gekennzeichnet, daß** die Apertur der Detektionseinrichtung kleiner 0,25 und größer 0,1 ist.

2. Navigationsvorrichtung (1) nach Anspruch 1, wobei die Detektionseinrichtung (10) einen konfokalen Detektor und/ oder einen Detektor auf Basis der Optischen Kohärenztomographie und/oder eine rotierende Spalt-Scheimpflugkamera und/oder einen konfokalen Laserscanner und/oder ein Ultraschallgerät und/oder eine Einrichtung zur dynamischen Wellenfrontdiagnose und/oder ein System zur dynamischen Stimulation der Aberrometrie und/oder ein Aberrometer und/oder ein Refraktometer aufweist.

3. Navigationsvorrichtung (1) nach einem der vorhergehenden Ansprüche, wobei die Apertur der Detektionseinrichtung (10) kleiner 0,22 und größer 0,18 ist.

4. Navigationsvorrichtung (1) nach einem der vorhergehenden Ansprüche, wobei die Detektionseinrichtung (10) eine polarisationsoptische Sensorik (15) aufweist.

5. Navigationsvorrichtung (1) nach einem der vorhergehenden Ansprüche, wobei die Bearbeitungseinrichtung (20) einen Laser, insbesondere einen fs-Laser, insbesondere einen Laser mit einem gescannten, fokussierten, ultrakurz gepulsten Laserstrahl aufweist.

6. Navigationsvorrichtung (1) nach einem der vorhergehenden Ansprüche, wobei die Bearbeitungseinrichtung (20) so ausgelegt ist, um Strahlung im optischen Element (50) zu erzeugen, die subdisruptiv mit Pulsenergien kleiner gleich 1 mikroJ stimuliert und/ oder disruptiv mit Pulsenergien größer 0,1 mikroJ perforiert und/ oder durch enge Aneinanderreihung der durch Disruption erzeugten Blasen schneidet.

7. Navigationsvorrichtung (1) nach einem der vorhergehenden Ansprüche, wobei das optische Element (50) eine Linse, insbesondere eine gealterte Augenlinse umfasst.

8. Navigationsvorrichtung (1) nach einem der vorhergehenden Ansprüche, wobei die Navigatiönsvorrichtung (1) eine ophtalmologische Saug-Spüleinrichtung (30) mit mindestens einer Kanüle (35) umfasst.

9. Navigationsvorrichtung (1) nach Anspruch 8, wobei die Saug-Spülvorrichtung (30) eingerichtet ist, nur Teile aus der inneren Struktur (55) des optischen Elements (50) zu entfernen, die vorher durch die Navigationsvorrichtung (1) bestimmt und durch die Bearbeitungseinrichtung (20) bearbeitet wurden.

10. Navigationsvorrichtung (1) nach einem der Ansprüche 8 bis 9, wobei die Vorrichtung eingerichtet ist, einen inneren Kern des optischen Elements (50) zu zerkleinern und herauszunehmen und den freigeräumten Bereich durch ein Gel aufzufüllen.

11. Therapieplanungs-Vorrichtung zur Planung der Therapie eines humanen Auges umfassend
ein dynamisches Wellenfrontmeßgerät zur Ermittlung von Wellenfrontdaten und
ein Diagnostik Gerät in einer Navigationsvorrichtung nach einem der vorhergehenden Ansprüche zur Bestimmung von geometrischen Parametern des Optikapparates des Auges und
eine Steuereinrichtung zur konsistenten Überlagerung der ermittelten Wellenfrontdaten und der Geometriedaten und eine weitere Steuereinrichtung zur Planung der effizientesten therapeutischen Laserschnittführung.

## Claims

1. Navigation apparatus (1) for optically analyzing the inner structure (55) of an optical element (50) and for processing the optical element (50), comprising a detection device (10) and a processing device (20), which is set up to be guided along or in a fixed relationship to the analyzed inner structure (55) of the optical element (50), **characterized in that** the aperture of the detection device is smaller than 0.25 and larger than 0.1.

2. Navigation apparatus (1) according to claim 1, wherein the detection device (10) has a confocal detector and/or a detector based on optical coherence tomography and/or a rotating slit Scheimpflug camera and/or a confocal laser scanner and/or an ultrasonic device and/or a device for dynamic wavefront diagnosis and/or a system for dynamic stimulation of the aberrometry and/or an aberrometer and/or a refractometer.

3. Navigation apparatus (1) according to one of the preceding claims, wherein the aperture of the detection device (10) is smaller than 0.22 and larger than 0.18.

4. Navigation apparatus (1) according to one of the preceding claims, wherein the detection device (10) has a polarization optical sensor system (15).

5. Navigation apparatus (1) according to one of the preceding claims, wherein the processing device (20) has a laser, in particular an fs laser, in particular a laser with a scanned, focused, ultra-short pulsed laser beam.

6. Navigation apparatus (1) according to one of the preceding claims, wherein the processing device (20) is designed to produce radiation in the optical element (50) which stimulates subdisruptively with pulse energies less than or equal to 1 microJ and/or perforates disruptively with pulse energies greater than 0.1 microJ and/or cuts by closely concatenating the bubbles produced by disruption.

7. Navigation apparatus (1) according to one of the preceding claims, wherein the optical element (50) comprises a lens, in particular an aged eye lens.

8. Navigation apparatus (1) according to one of the preceding claims, wherein the navigation apparatus (1) comprises an ophthalmological suction/irrigation device (30) with at least one cannula (35).

9. Navigation apparatus (1) according to claim 8, wherein the suction/irrigation device (30) is set up to remove only parts from the inner structure (55) of the optical element (50), which were previously determined by the navigation apparatus (1) and processed by the processing device (20).

10. Navigation apparatus (1) according to one of claims 8 to 9, wherein the apparatus is set up to comminute and abstract an inner nucleus of the optical element (50) and to fill the cleared area with a gel.

11. Therapy-planning apparatus for planning the therapy of a human eye, comprising
a dynamic wavefront measurement device for detecting wavefront data and
a diagnostic device in a navigation apparatus according to one of the preceding claims for determining geometric parameters of the optical apparatus of the eye and
a control device for consistent superposition of the detected wavefront data and of the geometric data and a further control device for planning the most efficient therapeutic laser cutting guidance.

## Revendications

1. Dispositif de navigation (1) destiné à l'analyse optique de la structure intérieure (55) d'un élément optique (50) et au traitement de l'élément optique (50), comprenant un dispositif de détection (10) et un dispositif de traitement (20) conçu pour être guidé le long de la structure intérieure (55) analysée de l'élément optique (50) ou fixement par rapport à elle, **caractérisé en ce que** l'ouverture du dispositif de détection est inférieure à 0,25 et supérieure à 0,1.

2. Dispositif de navigation (1) selon la revendication 1, le dispositif de détection (10) comportant un détecteur confocal et/ou un détecteur basé sur la tomographie de cohérence optique et/ou une caméra Scheimpflug à fente tournante et/ou un scanner à laser confocal et/ou un appareil à ultrason et/ou un dispositif de diagnostic de surface d'onde dynamique et/ou un système de stimulation dynamique d'aberrométrie et/ou un aberromètre et/ou un réfractomètre.

3. Dispositif de navigation (1) selon l'une quelconque des revendications précédentes, l'ouverture du dispositif de détection (10) étant inférieure à 0,22 et supérieure à 0,18.

4. Dispositif de navigation (1) selon l'une quelconque des revendications précédentes, le dispositif de détection (10) comportant un système de détection par polarisation (15).

5. Dispositif de navigation (1) selon l'une quelconque des revendications précédentes, le dispositif de traitement (20) comportant un laser, notamment un laser fs, notamment un laser avec un rayon laser balayé, focalisé, à pulsation ultracourte.

6. Dispositif de navigation (1) selon l'une quelconque des revendications précédentes, le dispositif de traitement (20) étant conçu pour produire un rayonnement dans l'élément optique (50) stimulé de façon disruptive avec les énergies d'impulsion inférieures ou égales à 1 microJ, et/ou perforé de façon disruptive avec des énergies d'impulsion supérieures à 0,1 microJ et/ou coupé par une succession étroite des bulles produites par la disruption.

7. Dispositif de navigation (1) selon l'une quelconque des revendications précédentes, l'élément optique (50) comprenant une lentille, notamment une lentille oculaire vieillie.

8. Dispositif de navigation (1) selon l'une quelconque des revendications précédentes, le dispositif de navigation (1) comprenant un dispositif de rinçage et d'aspiration (30) ophtalmique avec au moins une canule (35).

9. Dispositif de navigation (1) selon la revendication 8, le dispositif de rinçage et d'aspiration (30) étant conçu pour retirer uniquement des parties hors de la structure intérieure (55) de l'élément optique (50) auparavant déterminées par le dispositif de navigation (1) et traitées par le dispositif de traitement (20).

10. Dispositif de navigation (1) selon l'une quelconque des revendications 8 à 9, le dispositif étant conçu pour réduire et retirer le noyau intérieur de l'élément optique (50) et pour remplir la zone libérée au moyen d'un gel.

11. Dispositif de planification thérapeutique destiné à planifier le traitement d'un oeil humain comprenant :
un appareil de mesure de surface d'onde dynamique pour déterminer des données de surface d'onde ; et
un appareil de diagnostic dans un dispositif de navigation selon l'une quelconque des revendications précédentes pour déterminer des paramètres géométriques de l'appareil optique de l'oeil ; et
un dispositif de commande pour superposer de façon cohérente les données de surface d'onde déterminées et les données géométriques ; et
un dispositif de commande supplémentaire destiné à planifier la découpe laser thérapeutique de la façon la plus efficace possible.
